(19) Europäisches Patentamt
European Patent Office
Office européen des brevets

(11) Veröffentlichungsnummer : **0 099 068**
**B1**

(12) # EUROPÄISCHE PATENTSCHRIFT

(45) Veröffentlichungstag der Patentschrift : **30.09.87**

(51) Int. Cl.⁴ : **C 07 F 9/10**, C 07 F 9/09, C 07 F 9/24, A 61 K 31/66

(21) Anmeldenummer : **83106626.1**

(22) Anmeldetag : **06.07.83**

(54) **Neue Glycerinderivate zur Synthese von Phospholipiden.**

**Verbunden mit 83902086.4/0112872 (europäische Anmeldenummer/Veröffentlichungsnummer) durch Entscheidung vom 04.06.86.**

(30) Priorität : **06.07.82 DE 3225213**
**27.10.82 DE 3239817**

(43) Veröffentlichungstag der Anmeldung : **25.01.84 Patentblatt 84/04**

(45) Bekanntmachung des Hinweises auf die Patenterteilung : **30.09.87 Patentblatt 87/40**

(84) Benannte Vertragsstaaten :
**AT BE CH DE FR GB IT LI LU NL SE**

(56) Entgegenhaltungen :
**EP-A- 0 036 583**
**EP-A- 0 070 433**
**EP-A- 0 072 940**
**EP-A- 0 092 190**
**EP-A- 0 100 499**
**DE-A- 2 619 686**
**DE-A- 3 141 472**
**FR-A- 2 081 546**
**US-A- 4 329 302**
**SYNTHESES, Nr. 5, Mai 1982, Seiten 399-402, Georg Thieme Verlag, Speyer am Rhein, DE. C. A. A. VAN BOECKEL et al.: "Synthesis of 2-0-acetyl-3-0-hexadecyl-sn-1-glycerylphosphorylcholine, the enantiomer of platelet-activating factor (PAF)"**
**CHEMICAL ABSTRACTS, Band 96, Nr. 21, 24. Mai 1982, Seite 35, Nr. 174024h, Columbus, Ohio, USA**
**CHEMICAL ABSTRACTS, Band 98, Nr. 4, 24. Januar 1983, Seite 342, Nr. 22276v, Columbus, Ohio, USA**
**Lipide Isoprenoide mit Steroiden, G. Thieme Verlag, Stuttgart, S. 135-137**

(73) Patentinhaber : **Max-Planck-Gesellschaft zur Förderung der Wissenschaften e.V.**
**Bunsenstrasse 10**
**D-3400 Göttingen (DE)**

(72) Erfinder : **Eibl, Hansjörg, Prof. Dr.**
**Steinweg 51**
**D-3406 Bovenden (DE)**

(74) Vertreter : **Weickmann, Heinrich, Dipl.-Ing. et al**
**Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel**
**Postfach 860820**
**D-8000 München 86 (DE)**

**EP 0 099 068 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Glycerinderivate zur Synthese von Phospholipiden, wie Diester-, Monoester-, Monoäther-, Äther/Ester- und Diäther-phospholipiden und entsprechenden Phosphorsäureamid-Analoga.

Glycerinphosphatide sind biologisch und technisch wichtige Substanzen. Die Isolierung reiner, einheitlicher Glycerinphosphatide aus natürlichen Geweben ist schwierig und kostspielig. Ihre Synthese ist vor allem dann problematisch, wenn Glycerinphosphatide hergestellt werden sollen, in denen Glycerin mit bestimmten Resten in bestimmter Stellung acyliert sein soll.

E. Cubero Robles et al (Rec. Trav. Chim. 86 (1976), 762 ; Biochim. Biophys. Acta 187 (1969), 520) beschreiben zum Beispiel eine Synthese von Glycerinphosphatiden mit gemischten Fettsäureresten durch Acylierung von 1-Palmitoyl-sn-glycerin-3-phosphorcholin und Fettsäureanhydriden in Gegenwart von $Na_2O$. Bei diesem Verfahren findet jedoch in hohem Maße ein Austausch der Acylgruppen statt, wodurch die Selektivität, d. h. ganz gezielte Einführung bestimmter Acylreste, in bestimmter Stellung stark vermindert wird (vgl. K. M. W. Keough, P. J. Davis, Biochemistry 18 (1979), 1453). Ein weiterer Nachteil dieses Verfahrens ist es, daß während der Acylierungsstufe in erheblichem Maße auch eine Wanderung des Phosphorsäurerests aus der 3- in die 2-Stellung erfolgt. Auch bei der Reacylierung von Monoacyl-glycerinphosphorsäureestern können Umlagerungen sowohl von Fettsäuren als auch von Phosphatresten auftreten (vgl. A. Plückthun und E. A. Dennis, Biochemistry (1982), 21, 1743-1750 ; H. Eibl, Chem. Phys. Lipids (1980), 26, 405-429 ; H. Eibl, Liposomes : From Physical Structure to Therapeutic Application : Chapter 2 : Phospholipid Synthesis (1981), 19-50 ; C. G. Knight, Ed., Elsevier, Amsterdam).

Der Erfindung liegt deshalb die Aufgabe zugrunde, Ausgangsprodukte und Wege zur Herstellung von Glycerinphosphatiden (Phospholipiden) bereitzustellen, bei denen genannten Probleme nicht auftreten und die eine definierte Verteilung von Acyl-, Alkyl- und Phosphatresten über die Positionen 1, 2 und 3 des Glycerinmoleküls erlauben. Diese Aufgabe wird mit der vorliegenden Erfindung gelöst.

Gegenstand der Erfindung sind Glycerinderivate der Formeln I, II und III

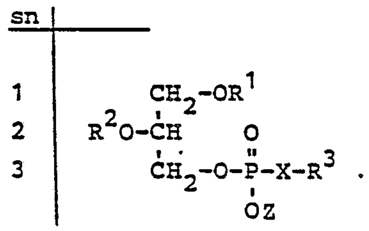
(I)

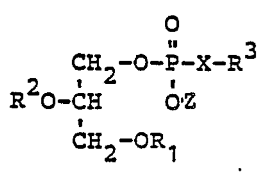
(II)

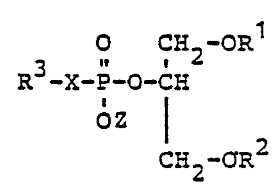
(III)

worin Z Methyl, gegebenenfalls einen Phenylrest enthaltendes Alkyl mit einer C—C-Mehrfachbindung in β-Stellung, Benzyl oder ein Äquivalent eines physiologisch verträglichen Kations, vorzugsweise Methyl, ist, $R^1$ und $R^2$ gleich oder verschieden sind und ein Wasserstoffatom, den Rest R oder den Acylrest —COR bedeuten, in 1- oder 3-Stellung auch Trityl, R Alkyl mit 1 bis 24 Kohlenstoffatomen oder Halogen-, Hydroxy- oder Carboxyalkyl mit 2 bis 24 Kohlenstoffatomen und R' Alkyl mit 9 bis 24 Kohlenstoffatomen oder Halogen-, Hydroxy- oder Carboxyalkyl mit 2 bis 24 Kohlenstoffatomen bedeutet, wobei Alkyl geradkettig oder verzweigt und Doppel- oder Dreifachbindungen enthalten kann, und durch Cycloalkyl mit 3 bis 6 Kohlenstoffatomen oder durch Arylreste substituiert sein kann, und worin eine Hydroxy- und/oder Carboxygruppe auch eine Schutzgruppe enthalten kann, X die Bedeutung O, NH oder $N(R^3)$ besitzt, und $R^3$, wenn X = O ist, Alkyl mit 1 bis 20 Kohlenstoffatomen oder Halogen-, Hydroxy- oder Carboxyalkyl oder auch Amino-, Methylamino-, Dimethylamino- oder Trimethylaminoalkyl mit 2 bis 20 Kohlenstoffatomen, in Formel I oder II, wenn $R^2$ ein Wasserstoffatom oder der Acylrest —COR' und Z ein Kation ist, aber nicht Halogenalkyl bedeutet, wobei Alkyl geradkettig oder verzweigt und Doppel- oder Dreifachbindungen enthalten kann, und durch Cycloalkyl mit 3 bis 5 Kohlenstoffatomen oder durch Arylreste substituiert sein kann, bedeutet, worin eine Hydroxy- und/oder Carboxygruppe auch eine Schutzgruppe enthalten kann, D- oder L-2-tert.-Butyloxycarbonylamino-2-tert.-butyloxy-carbonyl-äthyl, 1,2- oder 2,3-Isopropyliden-dioxy-propyl, 1,2-, 1,3- oder 2,3-Dibenzyloxy-propyl, 1,2,3,4,5-Pentabenzyloxycyclo-hexyl oder Aminoalkyl und N-Alkylaminoalkyl mit 2 bis 14 Kohlenstoffatomen in den Alkylresten, worin die Aminogruppen auch Schutzgruppen enthalten können, bedeutet, wenn X = NH ist, Alkyl mit 1 bis 20 Kohlenstoffatomen oder Halogen-, Hydroxy- oder Carboxyalkyl oder auch Amino-, Methylamino-, Dimethylamino- oder Trimethylammonioalkyl mit 2 bis 12 Kohlenstoffatomen, wobei Alkyl geradkettig oder verzweigt und Doppel- oder Dreifachbindungen enthalten kann, und durch Cycloalkyl mit 3 bis 6 Kohlenstoffatomen oder durch Arylreste substituiert sein kann, oder 1,2-Dihydroxypropyl bedeutet, worin eine Hydroxy- und/oder Carboxygruppe auch eine Schutzgruppe enthalten kann, und, wenn X = $N(R^3)$ ist, verschieden oder vorzugsweise gleich ist und Wasserstoff, Alkyl mit 1 bis 20 Kohlenstoffatomen oder Halogenalkyl mit 1 bis 12, vorzugsweise 2 bis 12 Kohlenstoffatomen, worin eine Alkylgruppe geradkettig oder verzweigt und Doppel- oder Dreifachbindungen enthalten kann und durch Cycloalkyl mit 3 bis 6 Kohlenstoffatomen oder durch Arylreste substituiert sein kann, bedeutet, mit der Maßgabe, daß in Formel

I oder II Z nicht Methyl bedeutet, wenn $R^1$ und $R^2$ einen unsubstituierten Alkylrest bedeuten, und ausgenommen das Natriumsalz von 1-Stearoyl-2-myristoyl-sn-glycero-3-phosphocholin ($Z = Na^+$).

Eine Alkylgruppe kann verzweigt oder vorzugsweise geradkettig sein und kann Doppel- oder Dreifachbindungen enthalten. Eine Alkylgruppe mit einer Doppelbindung ist zum Beispiel der als Schutzgruppe dienende Allylrest.

Cycloalkyl ist zum Beispiel Cyclopropyl, Cyclobutyl, Cyclopentyl, Methylcyclopentyl und vorzugsweise Cyclohexyl.

Ein Arylrest ist insbesondere ein solcher mit 6 bis 14 Kohlenstoffatomen, vorzugsweise Naphthyl-(1) oder -(2), und insbesondere Phenyl. Eine mit einem Phenylrest substituierte Alkyl-gruppe ist zum Beispiel die als Schutzgruppe dienende Benzylgruppe.

Eine Acylgruppe —COR′ leitet sich insbesondere von gesättigten und ungesättigten natürlichen Fettsäuren ab, wie zum Beispiel von Behen-, Laurin-, Stearin-, Palmitin-, Myristin-, Caprin- oder Arachinsäure, sowie von Öl-, Linol- oder Arachidonsäure und von höher ungesättigten Fettsäuren.

Halogen kann Fluor, Brom oder Jod sein, und ist insbesondere Chlor.

Ein physiologisch verträgliches Kation Z ist vorzugsweise ein ein-, zwei- oder dreiwertiges physiologisch verträgliches Kation, wie z. B. Natrium, Kalium, Calcium und insbesondere Lithium. Wenn Z alkyl bedeutet mit β-Unsättigung handelt es sich vorzugsweise und Alkyl oder dessen Homologe.

Schutzgruppen für die Hydroxygruppe sind zum Beispiel Benzyl, Allyl, Trityl, Tetrahydropyranyl, Mesyl und Tosyl ; für die Carboxygruppe zum Beispiel tert.-Butyl.

Die Vorstufen der Verbindungen der Formeln I, II oder III mit freien Hydroxygruppen am Phosphor können analog einem für Glycerinderivate bekannten Syntheseweg erhalten werden (vgl. zum Beispiel D. Arnold, H. U. Weltzien und O. Westphal, Liebigs Ann. Chem. 709 (1967), 234 bis 239 ; H. U. Weltzien und O. Westphal, Liebigs Ann. Chem. 709 (1967), 240 bis 243 ; H. Eibl. u. O. Westphal, Liebigs Ann. Chem. 709 (1967), 244 bis 247) vorzugsweise werden sie nach einem der in der deutschen Patentanmeldung P 3 130 867 der gleichen Anmelderin vom 4. August 1981 oder in der deutschen Patentanmeldung P 3 239 858 « Neue D-Mannitderivate als Ausgangsprodukte zur Synthese von Phospholipiden » der gleichen Anmelderin vom gleichen Anmeldetag für ähnliche Verbindungen bekannten Verfahren hergestellt. Die Überführung in den Methylester erfolgt dann in an sich bekannter Weise, zum Beispiel durch Umsetzung mit Methanol.

Die Herstellung der Glycerinderivate der Formeln I, II oder III, in denen Z ein Äquivalent eines physiologisch verträglichen Kations ist, kann insbesondere durch Umsetzung des entsprechenden Methylesters ($Z = CH_3$) mit einem Halogenid von Z, z. B. mit Lithiumbromid, erfolgen.

Es hat sich gezeigt, daß man ausgehend von den Zwischenprodukten der Formeln I, II oder III auf einfache und wirtschaftliche Weise Glycerinderivate mit verschiedenen Resten stellungsspezifisch und mit hoher Selektivität herstellen kann.

Die Verbindungen der Formel I führen zu Phospholipiden natürlicher Konfiguration (sn-3-Phosphate), die der Formel II und III zu Verbindungen mit nicht natürlicher Konfiguration (sn-1- oder -2-Phosphate).

Die vorstehend und nachstehend genannten Glycerinderivate können sowohl als optisch reine Stereoisomere als auch als Racemate vorliegen.

Bei zwei gleichkettigen Fettsäuren oder Alkylresten und einem Phosphatrest sind drei Stereoisomere möglich ; bei zwei ungleichen Fettsäuren oder Alkylresten und einem Phosphatrest sind sechs Stereoisomere möglich, die alle aus den Zwischenverbindungen I, II und III erhalten werden können.

Die Methylgruppe in Formel I, II und III ist in vieler Hinsicht entsprenchend Benzylgruppen überlegen. Sie besitzt ausreichende Stabilität für die notwendigen Reaktionsschritte, beispielsweise sind die Phosphorsäuremethylester der Formeln I, II und III stabil bei katalytischer Hydrogenolyse, saurer Trityl- und Propenylabspaltung unter den gewählten Bedingungen. Mit Lithiumbromid ist die Methylgruppe leicht und ohne Destruktion des Phospholipidmoleküls wieder entfernbar.

Gegenstand der Erfindung ist deshalb auch ein Verfahren zur Weiterverarbeitung der Verbindungen der Formel I, II oder III zur Herstellung von Phospholipiden, das dadurch gekennzeichnet ist, daß man

a) zur Herstellung von Diester-phospholipiden mit gleichen Acylresten von einer Verbindung ausgeht, in der $R^1$ und $R^2$ = Wasserstoff bedeutet, diese acyliert und danach mit Lithiumbromid demethyliert, oder

b) zur Herstellung von Diester-phospholipiden mit ungleichen Acylresten von einer Ausgangsverbindung ausgeht, in den $R^1$ und $R^2$ verschieden sind und $R^1$ Alkanoyl, Benzyl, Allyl oder Trityl ud $R^2$ Alkanoyl, Benzyl oder Allyl bedeutet, wobei ein Rest $R^1$ oder $R^2$ eine Alkanoylgruppe ist, eine Benzyl-, Allyl- oder Tritylgruppe $R^1$ oder $R^2$ abspaltet, die freiwerdende Hydroxylgruppe acyliert und danach mit Lithiumbromid demethyliert, oder

c) zur Herstellung von Monoester-phospholipiden von einer Ausgangsverbindung ausgeht, in der $R^1$ und $R^2$ verschieden sind und $R^1$ Alkanoyl, Benzyl, Allyl oder Trityl bedeutet, $R^2$ Alkanoyl, Benzyl oder Allyl, wobei ein Rest $R^1$ oder $R^2$ eine Alkanoylgruppe ist, und mit Lithiumbromid demethyliert, oder

d) zur Herstellung von Monoäther-phospholipiden von einer Verbindung ausgeht, in der $R^1$ und $R^2$ verschieden sind und $R^1$ Alkyl, Benzyl, Allyl oder Trityl bedeutet, $R^2$ Alkyl, Benzyl oder Allyl, wobei ein Rest $R^1$ oder $R^2$ eine Alkylgruppe ist, und mit Lithiumbromid demethyliert, oder

e) zur Herstellung von Äther/Ester-phospholipiden von einer Verbindung ausgeht, in der $R^1$ und $R^2$

verschieden sind und $R^1$ Alkyl, Benzyl, Allyl oder Trityl bedeutet, $R^2$ Alkyl, Benzyl oder Allyl, wobei ein Rest $R^1$ oder $R^2$ eine Alkylgruppe ist, eine Benzyl-, Allyl- oder Tritylgruppe $R^1$ oder $R^2$ abspaltet, die entstehende Hydroxygruppe acyliert, und danach mit Lithiumbromid demethyliert, oder

f) zur Herstellung von Diäther-phospholipiden von einer Verbindung ausgeht, in der $R^1$ und $R^2$ Alkyl sind, und mit Lithiumbromid demethyliert,

und wenn erforderlich oder erwünscht, in einer nach a) bis f) erhaltenen Verbindung auf an sich bekannte Weise vorhandene Schutzgruppen abspaltet und/oder den Phosphor enthaltenden Rest aminiert oder in einen anderen Phosphor enthaltenden Rest überführt, wobei die Abspaltung von Benzyl, Allyl oder Trityl auch nach der Aminierung oder Überführung in einen anderen Phosphor enthaltenden Rest erfolgen kann. Nach diesen Verfahren ist auch die Überführung einer Verbindung der Formel I, II oder III in eine andere Verbindung der Formel I, II oder III mit anderen Resten $R^1$, $R^2$, $R^3$ oder Z möglich.

Wenn erwünscht oder zweckmäßig, können die Verfahren auch so durchgeführt werden, daß man von einem nach einer der Verfahrensstufen erhaltenen Produkt ausgeht und die restlichen Verfahrensstufen durchführt. Unter Berücksichtigung aller im Molekül vorhandenen Reste ist es zum Beispiel auch möglich, die Reihenfolge der einzelnen Verfahrensstufen zu vertauschen.

Die Acylierung einer freien Hydroxygruppe kann durch Umsetzung mit Säurechloriden in Gegenward von Triäthylamin oder Pyridin durchgeführt werden (vgl. H. Eibl und O. Westphal, Liebigs Ann. Chem. 709 (1967), 244). Es ist aber auch möglich, nach an sich bekannten anderen Acylierungsmethoden (zum Beispiel wie von Gupta et al., Proc. Nat. Acad. Sci. USA 74 (1977), 4315 beschrieben) zu arbeiten. Besonders einfach gelingt die Acylierung mit freier Fettsäure und Dicyclohexylcarbodiimid in Gegenwart von Dimethylaminopyridin.

Die Abspaltung der Tritylgruppe erfolgt unter schwach sauren Bedingungen, vorzugsweise bei einem pH-Wert von 4 bis 6, wobei der günstigste Wert unter Berücksichtigung der übrigen Substituenten im Molekül leicht eruiert werden kann. Die Allyl- und Benzylschutzgruppen sind hierbei vollkommen stabil. Die Reaktion kann in einem wäßrigen oder wäßrig-organischen, aber auch in einem rein organischen Medium, wie zum Beispiel in absolutem Äthanol, in Gegenwart von HCl oder $H_2SO_4$ durchgeführt werden. Das organische Lösungsmittel kann dabei ein mit Wasser mischbares oder aber auch ein mit Wasser nur teilweise oder nur wenig mischbares inertes Lösungsmittel sein. Die Reaktion erfolgt, insbesondere beim Arbeiten in einem Zweiphasensystem, vorteilhafterweise unter starkem Rühren. Die Temperatur beträgt im allgemeinen 20 bis 80 °C. Zur Verbesserung der Löslichkeit kann es zweckmäßig sein, einen höheren Alkohol, wie zum Beispiel Propanol-(2), in kleiner Menge zuzusetzen.

Die Abspaltung der Benzylgruppe erfolgt durch katalytische Hydrogenolyse. Die Reaktionsbedingungen entsprechen dabei den üblichen Bedingungen. Insbesondere führt man die Hydrogenolyse in einem inerten Lösungsmittel, wie zum Beispiel Äthanol, in Gegenwart eines Palladium- oder Platin/Palladium-Katalysators durch, vorzugsweise bei Raumtemperatur und unter Normaldruck (vgl. H. Eibl et al, Liebigs Ann. Chemie, 738 (1970), 161).

Die Abspaltung der Allylgruppe (Umlagerung in Propenyl und nachfolgende Abspaltung von Propenyl) kann nach zwei verschiedene Methoden erfolgen, nämlich 1) unter alkalischen Bedingungen, wie zum Beispiel mit Kalium-tert.-butylat in Dimethylformamid und anschließende Spaltung mit Brom in gepufferter Lösung bei einem pH-Wert um 5 bis 6, oder 2) durch Umlagerung in Gegenwart eines Palladium-(Kohle)-Katalysators unter Bildung der unter diesen Bedingungen instabilen, spontan abspaltenden Propenylgruppe, wobei zweickmäßigerweise in 80 %-igem Methanol, welches in der wäßrigen Phase 20 % Ameisensäure enthält, bei Rückflußtemperatur gearbeitet wird. Im allgemeinen ist die Variante 1, d. h. die Abspaltung mit Brom, bevorzugt. Zur Abspaltung der Propenylgruppe in 1-Stellung kann auch Jod verwendet werden (Eibl und Lands, Biochemistry 9 (1970), 423). Während aber eine Abspaltung der Propenylgruppe in 2-Stellung mit Jod überhaupt nicht möglich ist, läßt sich eine solche Abspaltung überraschenderweise mit Brom vollständig und in wenigen Minuten durchführen.

Die Demethylierung des Phosphorsäuremethylesters mit Lithiumbromid erfolgt durch Kochen in einem geeigneten organischen Lösungsmittel, vorzugsweise in Methyläthylketon.

Die Aminierung des Phosphor enthaltenden Restes kann in an sich bekannter Weise erfolgen (vgl. z. B. H. Eibl u. A. Nicksch, Chem. Phys. Lipids, 22 (1978), 1 ; W. Diembeck und H. Eibl, Chem. Phys. Lipids, 24 (1979), 237), ebenso die nachträgliche Alkylierung einer freien Aminogruppe.

Erfindungsgemäßen Verbindungen der Formel I und II kommt auch eine große Bedeutung aufgrund ihrer besonderen Wirksamkeit auf das Wachstum von Tumoren zu. Es wurde gefunden, daß insbesondere die Glycerinderivate der allgemeinen Formel I und II, in denen $R^1$ den Rest $-(CH_2)_m-CH_3$, worin m 13-19 ist, bedeutet, $R^2$ ein Wasserstoffatom und, wenn X = O ist, $R^3$ Alkyl mit 1 bis 12 Kohlenstoffatomen, durch Hydroxy oder Trityloxy ω-substituiertes Alkyl mit 2 bis 11 Kohlenstoffatomen, durch Carboxy oder tert.-Butyloxycarbonyl ω-substituiertes Alkyl mit 1 bis 11 Kohlenstoffatomen, 2-(Amino- oder tert.-Butyloxycarbonylamino)-2-tert.-butyloxycarbonyl-äthyl, oder durch Brom, Amino, Methylamino, Dimethylamino, Trimethylammonio, tert.-Butyloxycarbonylamino, tert.-Butyloxycarbonyl-methylamino ω-substituiertes Alkyl mit 3-11 Kohlenstoffatomen bedeutet, wenn X = NH ist, $R^3$ Alkyl mit 1 bis 12 Kohlenstoffatomen, durch Brom, Hydroxy, Trityloxy, Amino, Methylamino, Dimethylamino oder Trimethylammonio ω-substituiertes Alkyl mit 2-11 Kohlenstoffatomen, durch Carboxy oder tert.-Butyloxycarbonyl ω-substituiertes Alkyl mit 1 bis 11 Kohlenstoffatomen oder 1,2-Dihydroxy- oder 1,2-Isopropylidendioxy-propyl bedeutet, und,

wenn X = N($R^3$) ist, $R^3$ verschieden oder vorzugsweise gleich ist und Alkyl mit 2 bis 4 Kohlenstoffatomen oder ω-Chloralkyl mit 1 bis 4 Kohlenstoffatomen bedeutet, eine ausgeprägte tumorwachstumshemmende Wirkung besitzen. Diese Wirkung zeigen sowohl die optisch reinen Verbindungen als auch die Racemate. Eine besonders gute Wirkung zeigen Verbindungen der Formel II und insbesondere I, in denen $R^1$ n-Octadecyl, $R^2$ Wasserstoff, X = O und $R^3$ 3-Trimethylammoniopropyl bedeuten.

Die ausgeprägte tumorwachstumshemmende Wirkung der erfindungsgemäßen Lyso-Verbindungen (1-Alkyl-2-OH) beruht zum Teil auf einem völlig neuen Wirkprinzip. Sie werden durch Acyltransferasen nicht oder nur sehr langsam entgiftet. Dies beruht bei einigen der erfindungsgemäßen Lysoverbindungen, bei denen der Rest $R^3$ z. B. eine durch Amino- oder alkylsubstituiertes Amino ω-substituierte Alkylgruppe mit mehr als 3 Kohlenstoffatomen ist, darauf, daß zwischen P und N mindestens 3 Methylengruppen liegen, wie z. B. in der Gruppierung

$$-O-\underset{\substack{| \ominus \\ O}}{P(O)}-O-(CH_2)_3-\overset{\oplus}{N}(CH_3)_3.$$

In einem solchen Fall greifen Acyltransferasen nicht mehr an ; Phosphorlipasen $A_2$ greifen noch an, die Acyltransferasen können aber nicht mehr reacylieren.

Die Verbindungen der Formeln I und II, in denen $R^1$ den Rest $-(CH_2)_m-CH_3$, worin m 13 bis 19 ist, und $R^2$ einen Acylrest COR′ bedeutet, wirken als in natürlicher Form vorliegende Vorläufer der vorstehend genannten tumorwirksamen Substanzen. Sie gehen mit Phospholipase $A_2$ in vivo in die aktiven Substanzen über.

Die 1-Alkyl-2-acyl-glycerinderivate sind im Gegensatz zu den 1-Alkyl-2-OH-Verbindungen, in die sie erst enzymatisch überführt werden, nicht hämolytisch ; sie können deshalb problemlos höher dosiert werden. Dabei ist es günstig, Matrixmoleküle mit stabilen Ätherstrukturen zu verwenden, die durch Phospholipase $A_2$ nicht gespalten werden, wie insbesonders z. B. 1-Octadecyl-2-oleyl-Verbindungen.

Die Wirksamkeit von Verbindungen auf das Wachstum von Tumoren wird zweckmäßig an Tumoren in Versuchstieren bewiesen. Hierfür kommen verschiedene experimentelle Tumoren zur Verwendung, beispielsweise der Ehrlich-Ascites-Tumor, ein Methylcholanthren-induzierter Tumor und ein Myelom-Tumor in Mäusen, ferner ein chemisch induzierter Rattentumor. Die Anti-Tumor-Substanzen werden parenteral in die tumortragenden Versuchstiere verabreicht. Bevorzugt wird die intravenöse und die intra- bzw. subkutane Applikation. Auch die orale Applizierbarkeit ist bei entsprechend höherer Dosierung des Anti-Tumormittels und bei einer physiologisch verträglichen Zubereitung, z. B. in Kapseln, nicht ausgeschlossen.

Gegenstand der Erfindung sind deshalb auch Arzneimittel, die eine oder mehrere der oben genannten tumorwachstumshemmenden Verbindungen der Formel I oder II als Wirkstoff enthalten. Neben den üblichen pharmazeutischen Konfektionierungs- und/oder Verdünnungsmitteln können diese Arzneimittel neben den Verbindungen der Formel I und II zur Unterstützung der Therapie gegebenenfalls auch noch weitere Wirkstoffe enthalten, sofern diese mit den erfindungsgemäßen Verbindungen zusammen keine unerwünschten Nebenwirkungen zeigen. Die Dosierungs- und Anwendungsweise entspricht im wesentlichen den für die Anti-Tumormittel der DE-OS 2 619 686 angegebenen Bedingungen, wobei aber aufgrund der höheren Wirksamkeit auch geringere Dosierungen und/oder eine weniger häufige Verabreichung in Frage kommen können. Als Dosierung hat es sich bei der parenteralen Applikation zweckmäßig erwiesen, etwa 0,05 bis 5 mg/kg Körpergewicht einzusetzen. Um die Anti-Tumormittel über längere Zeit im Kreislauf persistieren zu lassen, ist es häufig sinnvoll, die Mittel täglich oder in 2- bis 3-tägigen Abständen zu applizieren.

Als besonders günstige Arzneimittelzusammensetzung hat sich eine solche erwiesen, bei der die erfindungsgemäße Wirkstoffkomponente aus einer Mischung aus ca. 80-95 Gew.-% 1-Alkyl-2-acyl-Verbindung und ca. 20-5 Gew.-% 1-Alkyl-2-OH-Verbindung besteht. Eine solche Zusammensetzung ist aufgrund des höheren Gehalts an 1-Alkyl-2-anyl-Verbindung nicht hämolytisch.

In den nachfolgenden Schemen A, B und C sind die Möglichkeiten der Variation im Phosphor enthaltenden Rest (polarer Bereich) aufgezeigt, ohne sie darauf zu beschränken (A : Phospholipide ; B und C : Phosphatidsäureamide). Ausgehend von den Triphosphosäureestern (A) oder den entsprechenden Phosphorsäureamiden (B, C) können alle wesentlichen Phospholipide natürlicher Membranen oder deren Analoga auf einfache Weise dargestellt werden.

Durch das erfindungsgemäße Verfahren werden die bekannten Nachteile der Synthese von Glycerinphosphatiden, insbesondere die Wanderung der Acylreste und/oder des Phosphorsäurerestes, vermieden. Es hat sich gezeigt, daß die Verbindungen der Formeln I, II und III sehr gute Ausgangsmaterialien zur Herstellung von Glycerinphosphatiden sind, wobei jeder einzelne Verfahrensschritt unter Bedingungen durchgeführt werden kann, durch die die übrigen Substituenten nicht beeinflußt werden.

Zusammenfassend kann also festgestellt werden, daß das erfindungsgemäße Verfahren eine sehr selektive, einfache und wirtschaftliche Methode zur Herstellung von Phospholipiden darstellt, die vor allem für die stereo- und stellungsspezifische Darstellung von Glycerinphosphatiden mit zwei oder drei verschieden Resten von Bedeutung ist.

Die nachfolgenden Beispiele erläutern die Erfindung weiter, ohne sie darauf zu beschränken.

Schema A

Diester-, Monoester-, Äther/Ester-, Monoäther- und Diätherphospholipide (R = Glyceringrundkörper mit Resten $R_1$ und $R_2$; X = O).

(1)

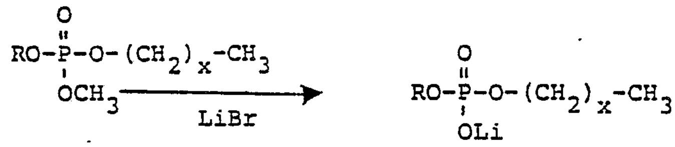


Phosphatidylalkylester
(x = 0 - 20)

(2)

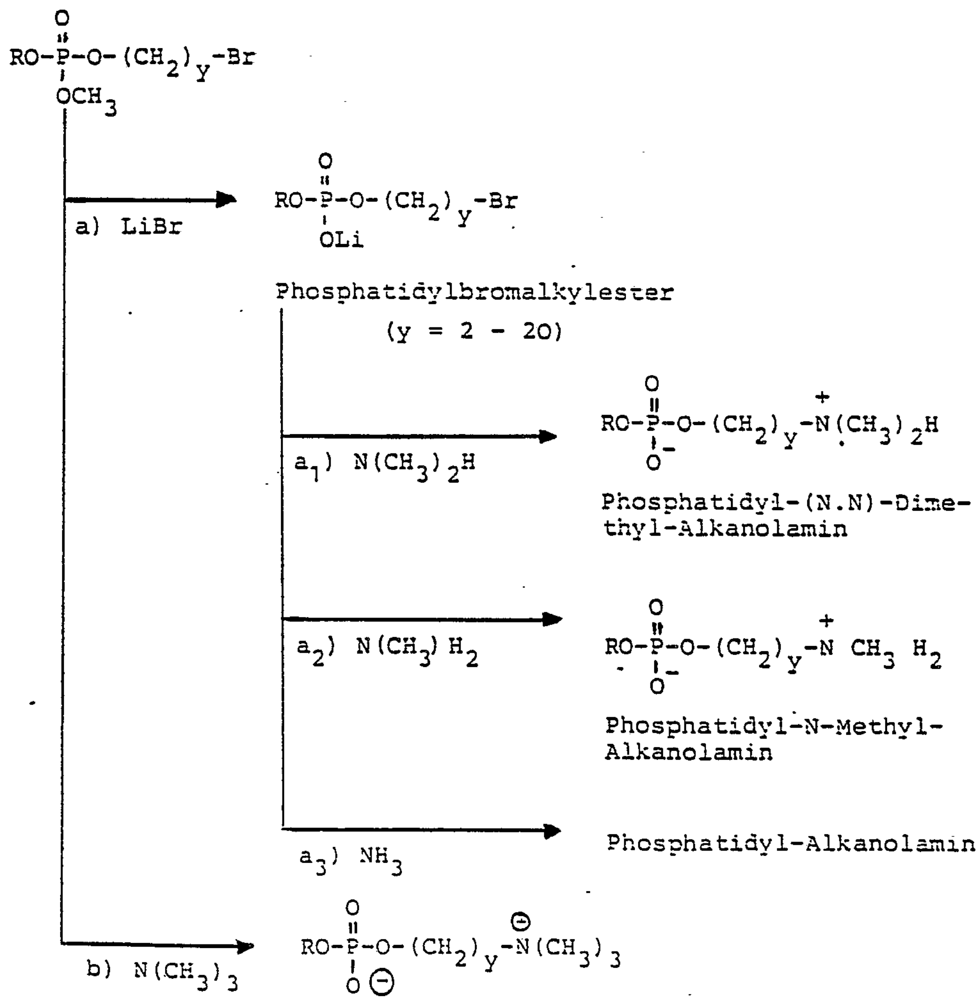


Phosphatidylbromalkylester
(y = 2 - 20)

Phosphatidyl-(N.N)-Dimethyl-Alkanolamin

Phosphatidyl-N-Methyl-Alkanolamin

Phosphatidyl-Alkanolamin

Phosphatidylcholin

(3)

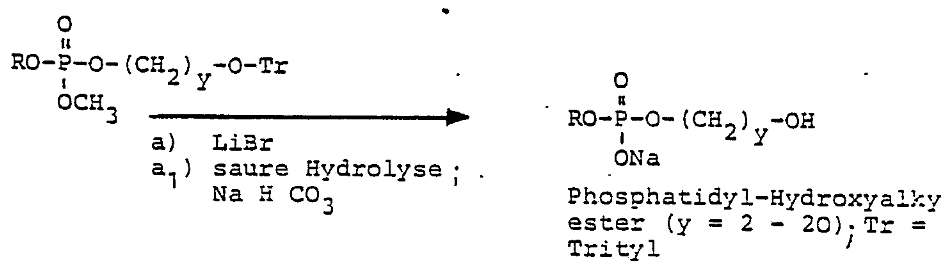


Phosphatidyl-Hydroxyalkylester (y = 2 - 20); Tr = Trityl

Schema A (Fortsetzung)

(4) 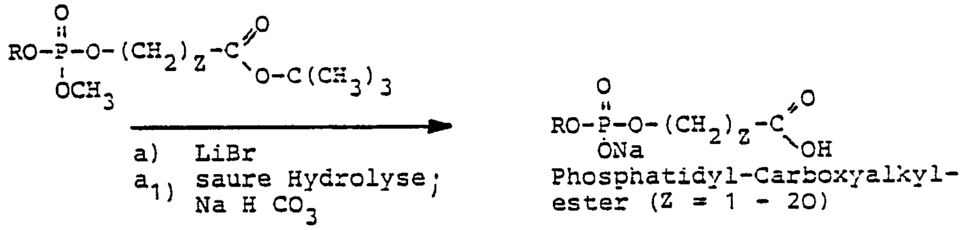


Phosphatidyl-Carboxyalkylester (Z = 1 - 20)

(5) 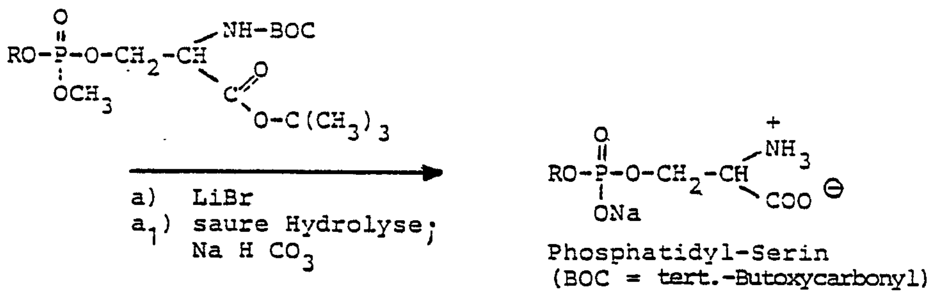


Phosphatidyl-Serin
(BOC = tert.-Butoxycarbonyl)

(6) 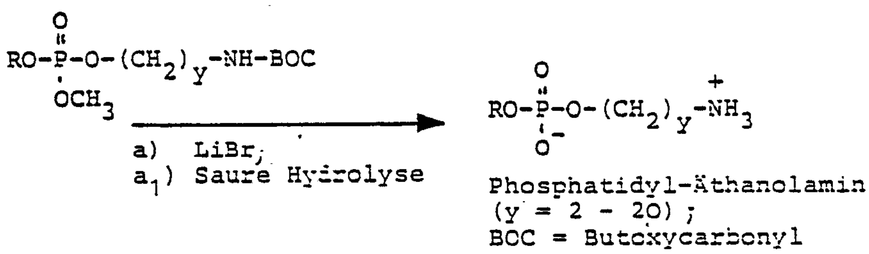


Phosphatidyl-Äthanolamin
(y = 2 - 20);
BOC = Butoxycarbonyl

(7) 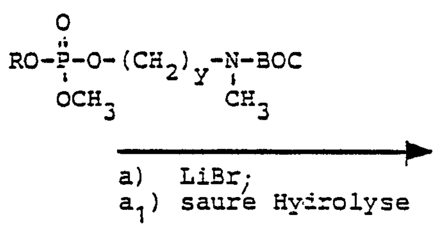


Phosphatidyl-N-Methyl-äthanolamin (y = 2 - 20)
BOC = Butoxycarbonyl

Schema B

Diester-, Monoester-, Äther/Ester-, Monoäther- und Diätherglycerinphosphorsäureamide (R = Glyceringrundkörper mit den Resten $R^1$ und $R^2$; X = NH).

(1) 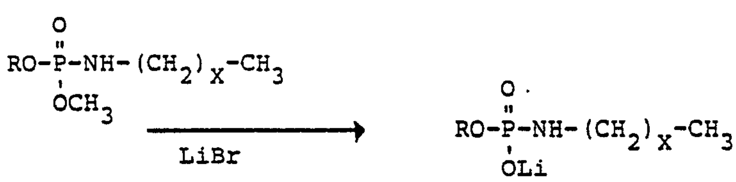


Phosphatidsäureamide
(X = 0-20)

Schema B (Fortsetzung)

(2)

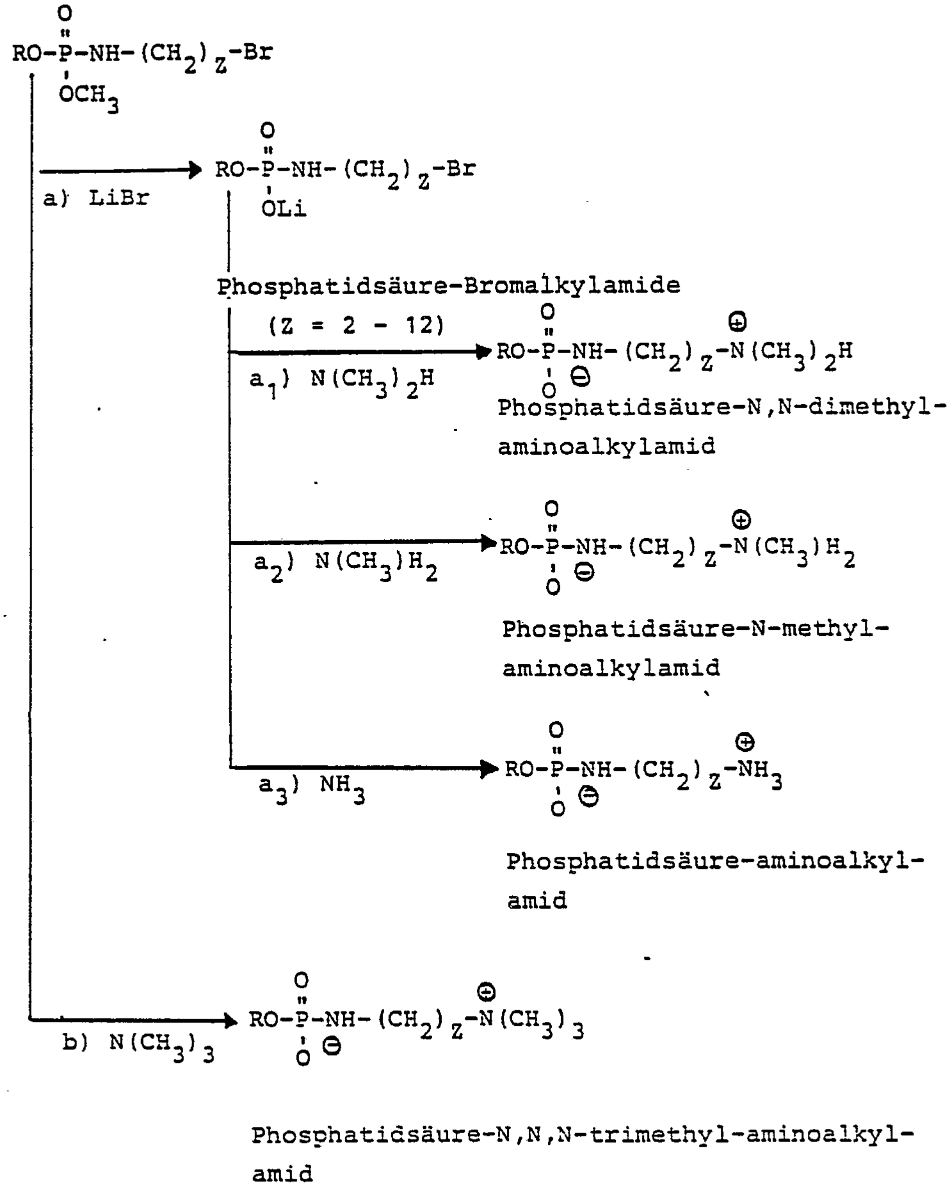


(3)

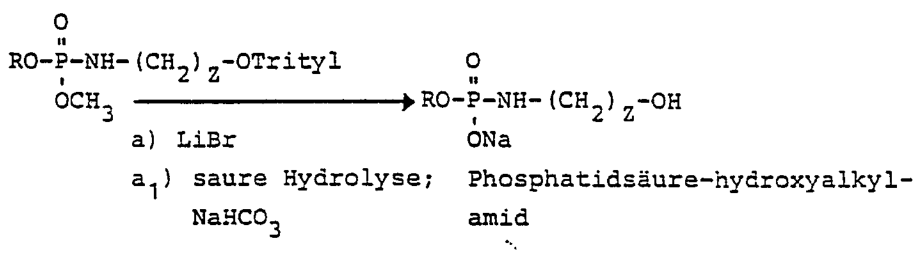


(4)

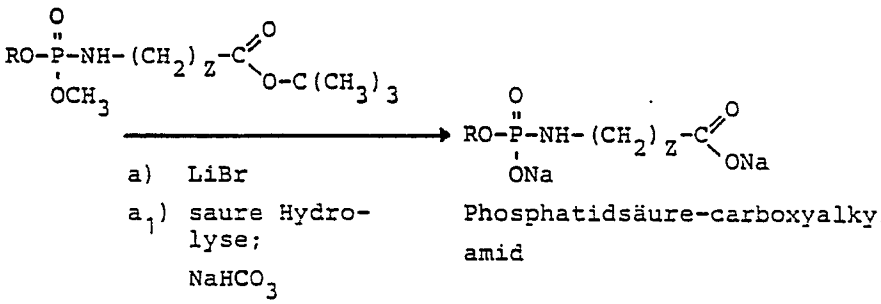

Schema B (Fortsetzung)

(5)

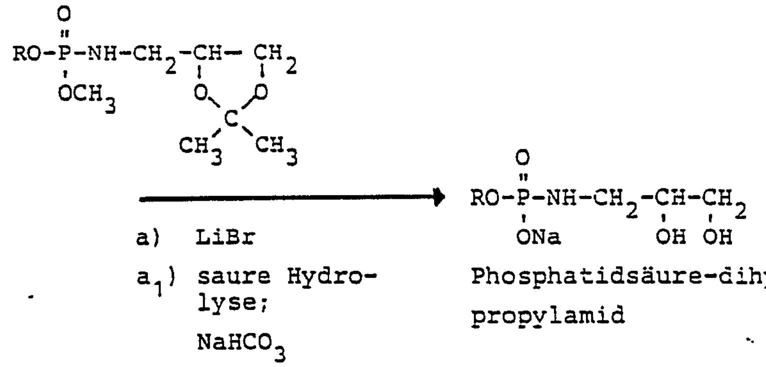


Phosphatidsäure-dihydroxypropylamid

Schema C

Diester-, Monoester-, Äther/Ester-, Monoäther- und Diätherglycerin-phosphorsäuredialkylamide (R = Glyceringrundkörper mit den Resten $R^1$ und $R^2$; X = $N(R^3)$).

(1)

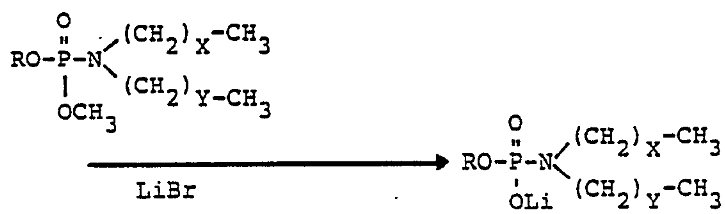


Phosphatidsäure-N,N-dialkylamid

(2)

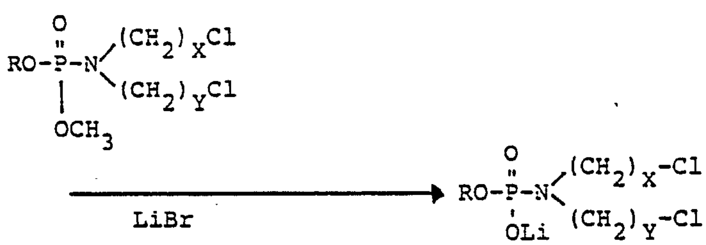


Phosphatidsäure-N,N-bis-(chloräthyl)-amid

## Beispiele

Diester-Phospholipide

gleichkettig

a) 1.2-Dibenzyl-sn-Glycerin

Ausgangsprodukt ist 3.4-Isopropyliden-D-Mannit (C.F. Wiggins, J. Chem. Soc. 13, 1946). Eine Lösung von 0.1 Mol in 1 l Toluol wird mit 0.5 Mol K-tert. Butylat versetzt und unter Rückfluß gekocht. Man tropft unter Rühren 0.5 Mol Benzylchlorid ein. Nach Abschluß der Reaktion (DC-Kontrolle) wird das Reaktionsgemisch mit Wasser extrahiert und die Toluolphase einrotiert. Der Rückstand wird zur Deacetonierung in 1 l Methanol/2-Propanol 1 : 1 (v/v) aufgenommen, mit 50 ml 2N—$H_2SO_4$ versetzt und unter Rückfluß gekocht. Nach Vollständigkeit der Reaktion (DC-Kontrolle) wird mit 1 l Diisopropyläther versetzt und zweimal mit 1 l Wasser gewaschen. Die Diisopropylätherphase wird einrotiert und das Zwischenprodukt, 1.2.5.6-Tetrabenzyl-D-Mannit wird chromatographisch an Kieselgel gereinigt. Die Ausbeuten liegen bei 90-95 %.

1.2.5.6-Tetrabenzyl-D-Mannit

($C_{34}H_{38}O_6$ ; 542,68)
ber : C 75.25 H 7.06
gef : C 75.11 H 6.99

1.2.5.6-Tetrabenzyl-D-Mannit (0.1 Mol) wird in 500 ml Benzol gelöst und portionsweise mit Bleitetraacetat (ca. 0.1 Mol) versetzt, bis das Ausgangsprodukt vollständig umgesetzt ist (DC-Kontrolle). Man wäscht zweimal mit jeweils 500 ml Wasser, rotiert die Benzolphase ein und nimmt den Rückstand in 500 ml Methanol auf. Die Lösung der Aldehyde in Methanol wird portionsweise mit $NaBH_4$ (ca. 0.1 Mol) versetzt. Nach Beendigung der Reaktion wird mit 500 ml Diisopropyläther versetzt und mit Wasser gewaschen. Die Diisopropylätherphase wird einrotiert und der Rückstand an Kieselgel chromatographiert. Die Ausbeute an 1.2-Dibenzyl-sn-glycerin beträgt 95 % ; Drehwert $\alpha_D^{20}$ = — 0,48 (in Substanz)

1.2-Dibenzyl-sn-Glycerin

($C_{17}H_{20}O_3$ ; 272,35)
ber : C 74,97 H 7,40
gef : C 74,91 H 7,40

b) Phosphorylierung

1.2-Dibenzyl-sn-Glycerin (0.1 Mol) werden in 500 ml THF gelöst und mit Triäthylamin (0.2 Mol) versetzt. Man tropft die Lösung unter starkem Rühren bei 15 °C zu Phosphoroxychlorid (0.15 Mol) in 100 ml THF. Nach Beendigung der Reaktion (DC-Kontrolle) wird vom ausgefallenen Triäthylaminhydrochlorid abfiltriert, mit 100 ml Toluol versetzt und im Vakuum bei 40 °C einrotiert (Entfernung von überschüssigem $POCl_3$). Der ölige Rückstand besteht hauptsächlich aus 1.2-Dibenzyl-sn-Glycero-3-Phosphorsäuredichlorid, das in 500 ml THF gelöst und tropfenweise mit Triäthylamin (0.3 Mol) und dann mit den entsprechenden Alkoholen, z. B. N-BOC-Serin-tert.-Butylester (0,15 Mol) (BOC = Butyloxycarbonyl) versetzt wird. Nach Beendigung der Reaktion wird mit Methanol (1 Mol) versetzt und nach vollständiger Reaktion aufgearbeitet. Man gibt 500 ml Diisopropyläther zu und extrahiert die organische Phase mit Wasser. Die Diisopropylätherphase wird einrotiert und der Rückstand an Kieselgel chromatographiert. Man erhält 1.2-Dibenzyl-sn-Glycero-3-Phospho-Methyl-N-BOC-Serin-tert. Butylester in Ausbeuten von 80 %.

1.2-Dibenzyl-sn-Glycero-3-Phospho-Methyl-N-BOC-Serin-tert. Butylester

($C_{30}H_{44}NO_{10}P$ ; 609,67)
ber : C 59,10 H 7,28 N 2,30 P 5,08
gef : C 58,95 H 7,31 N 2,17 P 5,01

c) Katalytische Debenzylierung

1.2-Dibenzyl-sn-Glycero-3-Phospho-Methyl-N-BOC-Serin-tert. Butylester (0.1 Mol) werden in 400 ml THF gelöst und mit 10 g Pd/C (10 % Pd auf Aktivkohle) und 1 g Palladiumschwarz versetzt. Man beläßt unter Rühren solange unter $H_2$-Atmosphäre, bis die $H_2$-Aufnahme abgeschlossen ist. Die Reaktion läuft quantitativ ab. Man filtriert den Katalysator ab und entfernt das Lösungsmittel im Vakuum. Der ölige Rückstand wird direkt weiter umgesetzt.

sn-Glycero-3-Phospho-Methyl-N-BOC-Serin-tert. Butylester

($C_{16}H_{32}NO_{10}P$ : 429,415)
ber : C 44,75 H 7,51 N 3,26 P 7,21
gef : C 44,59 H 7,39 N 3,14 P 7,12

Durch entsprechende Umsetzung mit anderen Alkoholen können weitere zentrale Zwischenprodukte erhalten werden, zum Beispiel :

sn-Glycero-3-Phospho-Methyl-Bromoäthylester
($C_6H_{14}BrO_6P$ ; 269,07)
ber : C 26,78 H 5,25 P 11,51
gef : C 26,63 H 5,21 P 11,01

d) Acylierung

Die Zwischenprodukte mit zwei freien Hydroxylgruppen in sn-1 und sn-2-Position des Glycerins können nun mit den gewünschten Fettsäuren umgesetzt werden, beispielsweise mit Palmitinsäure oder mit Ölsäure. Dazu werden 0.1 Mol in 300 ml Methylenchlorid gelöst, mit den Fettsäuren (0.25 Mol) in 200 ml Methylenchlorid, mit Dicyclohexylcarbodiimid (0.25 Mol) und 4-Dimethyl-Aminopyridin (0.05 Mol) versetzt. Unter Rühren bei 20 °C wird nach Abschluß der Reaktion vom ausgefallenen Harnstoff abfiltriert, mit Wasser gewaschen und die Methylenchloridphase einrotiert. Der Rückstand wird an Kieselgel durch Chromatographie gereinigt. Die Ausbeute an acyliertem Produkt beträgt 95 %.

1.2-Dioleoyl-sn-Glycero-3-Phospho-Methyl-N-BOC-L-Serin-tert. Butylester

($C_{52}H_{96}NO_{10}P$ ; 926,32)
ber : C 65,17 H 10,10 N 1,46 P 3,23
gef : C 64,86 H 10,11 N 1,47 P 3,17

e) Demethylierung

Der Phosphorsäurediester mit dem geschützten Serin (0.1 Mol) wird mit einer Lösung von LiBr (0.5 Mol) in Äthylmethylketon, 500 ml, versetzt und 60 Min. unter Rückfluß gekocht. Die Spaltung ist quantitativ. Man versetzt mit Diisopropyläther und extrahiert mit Wasser. Die Diisopropylätherphase wird einrotiert und der Rückstand direkt weiter umgesetzt.

f) Abspaltung der Serinschutzgruppen

Der Rückstand aus e) (0.1 Mol) wird in 300 ml Methylenchlorid gelöst und bei 0 °C mit 150 ml Trifluoressigsäure versetzt. Nach Zusatz von 30 ml 70 % $HClO_4$ wird 60 Min. bei 0 °C gerührt. Die Spaltung ist vollständig. Man versetzt mit 300 ml Chloroform, 600 ml Wasser und 600 ml Methanol. Die Chloroformphase wird in 600 ml 1M $NaHCO_3$ versetzt. Nach Zusatz von 600 ml $CH_3OH$ wird kräftig geschüttelt und die Chloroformphase nach Phasenseparation einrotiert. Der Rückstand wird an Kieselgel chromatographiert. Die Ausbeute beträgt 95 % bezogen auf den Phosphorsäuretriester.

1.2-Dioleoyl-sn-Glycero-3-Phosphoserin

($C_{42}H_{77}NNaO_{10}P$ ; 810,05)
ber : C 62,28 H 9,58 N 1,73 P 3,82
gef : C 62,09 H 9,41 N 1,49 P 3,69

gemischtkettig

a) Phosphorylierung

1-Trityl-2-Benzyl-sn-Glycerin (0.1 Mol) wird in 500 ml THF gelöst und mit Triäthylamin (0.2 Mol) versetzt. Man tropft die Lösung bei 15 °C unter starkem Rühren zu Phosphoroxychlorid (0.15 Mol) in 100 ml THF. Nach Beendigung der Reaktion wird filtriert, mit 100 ml Toluol versetzt und im Vakuum bei 40 °C einrotiert (überschüssiges Triäthylamin und Phosphoroxychlorid werden abgezogen). Der Rückstand, hauptsächlich 1-Trityl-2-Benzyl-sn-Glycero-3-Phosphodichlorid, wird in 500 ml THF aufgenommen und mit Triäthylamin (0.3 Mol) versetzt. Man tropft in diese Lösung beispielsweise Bromalkanole (0.2 Mol) bei 30 °C ein und versetzt nach Beendigung der Reaktion mit Methanol (1 Mol). Die Methanolyse ist bei 30 °C rasch abgeschlossen (ca. 2 Std.). Man versetzt mit 500 ml Diisopropyläther und extrahiert die organische Phase mit Wasser. Die Diisopropylätherphase wird einrotiert.

b) Detritylierung

Der ölige Rückstant (0.1 Mol) aus a) wird in 500 ml $CH_3OH$, die 5 ml konz. $H_2SO_4$ enthalten, aufgenommen und auf 50 °C erwärmt. Die Reaktion ist nach 10 Min. vollständig, man kühlt auf 0 °C und filtriert den kristallinen Trityl-Methyläther ab. Nach Zusatz von Chloroform, 500 ml, wird mit 500 ml 1M $NaHCO_3$ gewaschen (pH der wässrigen Phase soll mindestens 5 betragen). Die Chloroformphase wird über $Na_2SO_4$ getrocknet und einrotiert.

c) 1. Acylierung

Der Rückstand aus a) wird in 500 ml Methylenchlorid aufgenommen und mit Palmitinsäure (0.25 Mol) versetzt. Nach Zusatz von Dicyclohexylcarbodiimid (0.25 Mol) und 4-Dimethyl-Aminopyridin (0.05 Mol) wird bei 20 °C für 3 Std. gerührt. Man filtriert vom ausgefallenen Harnstoff ab, wäscht das Filtrat mit Wasser und entfernt das Methylenchlorid im Vakuum. Der Rückstand wird an Kieselgel chromatographiert. Die Ausbeute beträgt 75 % bezogen auf 1-Trityl-2-Benzyl-sn-Glycerin.

d) Katalytische Debenzylierung

1-Palmitoyl-2-Benzyl-sn-Glycero-3-Phospho-Methyl-Bromoalkylester (0.1 Mol) wird in Diisopropyläther, 500 ml, gelöst und mit 10 g Pd/C-Katalysator (10 % Pd) und 1 g Palladiumschwarz versetzt. Unter $H_2$-Atmosphäre wird solange gerührt, bis die Wasserstoffaufnahme vollständig ist. Man erhält quantitativ 1-Palmitoyl-sn-Glycero-3-Phospho-Methyl-Bromoalkylester. Vom Katalysator wird abfiltriert, THF vom Filtrat abgezogen und der Rückstand in 500 ml Tetrachlorkohlenstoff aufgenommen.

e) Zweite Acylierung

Die Tetrachlorkohlenstofflösung des Phosphorsäuretriesters (0.1 Mol) wird mit Dicyclohexylcarbodiimid (0.25 Mol) und Ölsäure (0.25 Mol) versetzt. Nach Zusatz von 4-Dimethyl-Aminopyridin wird bei 20 °C solange gerührt, bis die Reaktion vollständig ist (DC-Kontrolle).

Man trennt den Niederschlag durch Filtration ab, extrahiert mit Wasser und engt die Tetrachlorkohlenstoffphase im Vakuum ein. Der Rückstand wird an Kieselgel chromatographiert. Man erhält 1-Palmitoyl-2-Oleoyl-sn-Glycero-3-Phospho-Methyl-Bromoalkylester in Ausbeuten von ca. 95 %.

f) Demethylierung

Der Phosphorsäuretriester (0.1 Mol) aus e) wird mit 0.5 Mol LiBr in 500 ml Äthylmethylketon versetzt und 60 Min. unter Rückfluß gekocht. Die Ausbeute an Li-Salz von 1-Palmitoyl-2-Oleoyl-sn-Glycero-3-Phospho-Bromoalkylester ist quantitativ.

g) Aminierung

Zur Aminierung der Bromoalkylester kann in an sich bekannter Weise verfahren werden (vgl. z. B. H. Eibl und A. Nicksch, Chem. Phys. Lipids 22 (1978) 1 ; W. Diembeck und H. Eibl, Chem. Phys. Lipids 24 (1979) 237). Die Umsetzung mit dem Amin, z. B. Trimethylamin, erfolgt z. B. in Chloroform/Propanol-(2) (1/1) als Lösungsmittel.

h) Direkte Aminierung der Phosphorsäuretriester

Die Phosphorsäuretriester (0.1 Mol) aus e) werden in 500 ml Chloroform gelöst und mit 500 ml 2-Propanol versetzt, das Trimethylamin (1 Mol) enthält. Nach 24 Std. bei 50 °C ist die Reaktion vollständig. Man zieht das Lösungsmittel ab und versetzt mit jeweils 500 ml Chloroform, Methanol und Wasser. Die Chloroformphase enthält 1-Palmitoyl-2-Oleoyl-sn-Glycero-3-Phospho-N.N.N-Trimethylalkanolaminester. Man entfernt das Lösungsmittel und reinigt durch Chromatographie. Die Ausbeuten betragen mehr als 90 %.

Monoester-Phospholipide

a) Demethylierung

Die Produkte der ersten Acylierung (gemischtkettig, Reaktion c)) werden mit LiBr demethyliert ; so wird beispielsweise 1-Palmitoyl-2-Benzyl-sn-Glycero-3-Phospho-Methyl-Bromoalkylester (0.1 Mol) mit LiBr (0.5 Mol) in 500 ml Äthylmethylketon unter Rückfluß gekocht. Nach 60 Min. ist die Umsetzung quantitativ. Die Aminierung kann wie vorstehend unter g) beschrieben erfolgen.

b) Katalytische Debenzylierung

1-Palmitoyl-2-Benzyl-sn-Glycero-3-Phospho-N.N.N-Trimethylalkanolaminester (0.1 Mol) werden in Alkohol, 1 l, gelöst und mit 10 g Pd/C-Katalysator (10 % Palladium auf Aktivkohle) und 1 g Palladiumschwarz versetzt. Man rührt bis zur Beendigung der Wasserstoffaufnahme. Die Debenzylierung erfolgt quantitativ. Man filtriert den Katalysator ab und entfernt das Lösungsmittel im Vakuum. Der Rückstand wird in 100 ml $CHCl_3$ aufgenommen und mit 400 ml Aceton gefällt. Der Niederschlag wird abgesaugt und getrocknet.

Monoäther-Phospholipide

Die Monoäther-Phospholipide können auf völlig gleiche Weise wie die Monoester-Phospholipide dargestellt werden. Als Ausgangsprodukt wird in diesem Falle beispielsweise 1-Octadecyl-2-Benzyl-sn-Glycerin verwendet.

Äther/Ester-Phospholipide

Man erhält Äther/Ester-Phospholipide, wenn man beispielsweise 1-Octadecyl-2-Benzyl-sn-Glycerin als Ausgangsprodukt verwendet und nach dem verfahren für Diester-Phospholipide, gemischtkettig, vorgeht.

Nach der Phosphorylierung a) erfolgt jedoch sofort die katalytische Debenzylierung d) und die zweite Acylierung e). Dann wird, wie dort beschrieben, weiterverfahren.

Diäther-Phospholipide

1,2-Diäther-sn-Glycerine können wie die 1,2-Diester-sn-Glycerine in die entsprechenden Phospholipide umgewandelt werden, wenn man wie unter Diester-Phospholipiden, gleichkettig, verfährt und die Verfahrensschritte b), Phosphorylierung, e), Demethylierung und f), Abspaltung der Schutzgruppen, anwendet.

**Patentansprüche**

1. Glycerinderivate der Formeln I, II und III

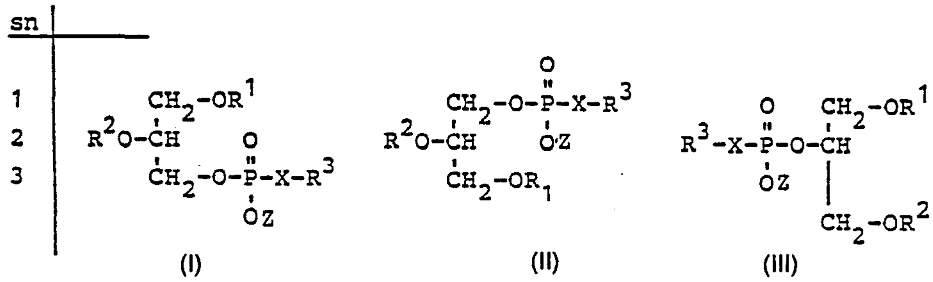

(I) (II) (III)

worin Z Methyl gegebenenfalls einen Phenylrest enthaltendes Alkyl mit einer C—C-Mehrfachbindung in β-Stellung, Benzyl oder ein Äquivalent eines physiologisch verträglichen Kations ist, $R^1$ und $R^2$ gleich oder verschieden sind und ein Wasserstoffatom, den Rest R oder den Acylrest —COR′ bedeuten, in 1- oder 3-Stellung auch Trityl, R Alkyl mit 1 bis 24 Kohlenstoffatomen oder Halogen-, Hydroxy- oder Carboxyalkyl mit 2 bis 24 Kohlenstoffatomen und R′ Alkyl mit 9 bis 24 Kohlenstoffatomen oder Halogen-, Hydroxy- oder Carboxyalkyl mit 2 bis 24 Kohlenstoffatomen bedeutet, wobei Alkyl geradkettig oder verzweigt und Doppel- oder Dreifachbindungen enthalten kann, und durch Cycloalkyl mit 3 bis 6 Kohlenstoffatomen oder durch Arylreste substituiert sein kann, und worin eine Hydroxy- und/oder Carboxygruppe auch eine Schutzgruppe enthalten kann, X die Bedeutung O, NH oder $N(R^3)$ besitzt, und $R^3$, wenn X = O ist, Alkyl mit 1 bis 20 Kohlenstoffatomen oder Halogen-, Amino-, Methylamino-, Dimethylamino-, Trimethylammonio-, Hydroxy- oder Carboxyalkyl mit 2 bis 20 Kohlenstoffatomen, in Formel I oder II, wenn $R^2$ ein Wasserstoffatom oder der Acylrest —COR′ und Z ein Kation ist, aber nicht Halogenalkyl bedeutet, wobei Alkyl geradkettig oder verzweigt und Doppel- oder Dreifachbindungen enthalten kann, und durch Cycloalkyl mit 3 bis 6 Kohlenstoffatomen oder durch Arylreste substituiert sein kann, bedeutet, worin eine Amino-, Hydroxy- und/oder Carboxygruppe auch eine Schutzgruppe enthalten kann, oder D- oder L-2-tert.-Butyloxycarbonylamino-2-tert.-butyloxycarbonyl-äthyl-, 1,2- oder 2,3-Isopropylidendioxy-propyl-, 1,2-, 1,3- oder 2,3-Dibenzyloxy-propyl-, 1,2,3,4,5-Pentabenzyloxy-cyclohexyl- oder N-Alkylaminoalkyl mit 2 bis 14 Kohlenstoffatomen in den Alkylresten, worin die Aminogruppen auch Schutzgruppen enthalten können, bedeutet, wenn X = NH ist, Alkyl mit 1 bis 20 Kohlenstoffatomen oder Halogen-, Amino-, Methylamino-, Dimethylamino-, Trimethylammonio-, Hydroxy- oder Carboxyalkyl mit 2 bis 12 Kohlenstoffatomen, wobei Alkyl geradkettig oder verzweigt und Doppel- oder Dreifachbindungen enthalten kann, und durch Cycloalkyl mit 3 bis 6 Kohlenstoffatomen oder durch Arylreste substituiert sein kann, oder 1,2-Dihydroxypropyl bedeutet, worin eine Amino-, Hydroxy- und/oder Carboxygruppe auch eine Schutzgruppe enthalten kann, und, wenn X = $N(R^3)$ ist, gleich oder verschieden ist und Wasserstoff, Alkyl mit 1 bis 20 Kohlenstoffatomen oder Halogenalkyl mit 1 bis 12 Kohlenstoffatomen bedeutet, worin eine Alkylgruppe geradkettig oder verzweigt und Doppel- oder Dreifachbindungen enthalten kann, und durch Cycloalkyl mit 3 bis 6 Kohlenstoffatomen oder durch Arylreste substituiert sein kann, mit der Maßgabe, daß in Formel I oder II Z nicht Methyl bedeutet, wenn $R^1$ und $R^2$ einen unsubstituierten Alkylrest bedeuten, und ausgenommen das Natriumsalz von 1-Stearoyl-2-myristoyl-sn-glycero-3-phosphocholin ($Z = NA^+$).

2. Glycerinderivate der Formel I oder II nach Anspruch 1, dadurch gekennzeichnet, daß $R^1$ den Rest $-(CH_2)_m-CH_3$, worin m 13-19 ist, und $R^2$ einen Acylrest COR′ bedeutet.

3. Glycerinderivate der Formel I oder II nach Anspruch 1, dadurch gekennzeichnet, daß $R^1$ den Rest $-(CH_2)_m-CH_3$, worin m 13-19 ist, bedeutet, $R^2$ ein Wasserstoffatom und, wenn X = O ist, $R^3$ Alkyl mit 1 bis 12 Kohlenstoffatomen, durch Hydroxy oder Trityloxy ω-substituiertes Alkyl mit 2 bis 11 Kohlenstoffatomen, durch Carboxy oder tert.-Butyloxycarbonyl ω-substituiertes Alkyl mit 1 bis 11 Kohlenstoffatomen, 2-(Amino- oder tert.-Butyloxycarbonylamino)-2-tert.-butyloxycarbonyl-äthyl, 1,2-Dihydroxy- oder 1,2-Isopropylidendioxy-propyl, Pentahydroxy- oder Pentabenzyloxy-cyclohexyl, oder durch Amino, Methylamino, Dimethylamino, Trimethylammonio, tert.-Butyloxycarbonylamino, tert.-Butyloxycarbonylmethylamino ω-substituiertes Alkyl mit 3-11 Kohlenstoffatomen bedeutet, wenn X = NH ist, $R^3$ Alkyl mit 1 bis 12 Kohlenstoffatomen, durch Brom, Hydroxy, Trityloxy, Amino, Methylamino, Dimethylamino oder Trimethylammonio ω-substituiertes Alkyl mit 2-11 Kohlenstoffatomen, durch Carboxy oder tert.-Butyloxycarbonyl ω-subst. Alkyl mit 1 bis 11 Kohlenstoffatomen oder 1,2-Dihydroxy- oder 1,2-Isopropylidendioxy-propyl bedeutet, und, wenn X = $N(R^3)$ ist, $R^3$ verschieden oder gleich ist und Alkyl mit 2 bis 4 Kohlenstoffatomen oder ω-Chloralkyl mit 1 bis 4 Kohlenstoffatomen bedeutet.

4. Verfahren zur Weiterverarbeitung der Verbindungen der Formeln I, II oder III nach einem der Ansprüche 1 bis 3 zur Herstellung von Phospholipiden, dadurch gekennzeichnet, daß man

a) zur Herstellung von Diester-phospholipiden mit gleichen Acylresten von einer Verbindung ausgeht, in der $R^1$ und $R^2$ = Wasserstoff bedeutet; diese acyliert und danach mit Lithiumbromid demethyliert, oder

b) zur Herstellung von Diester-phospholipiden mit ungleichen Acylresten von einer Ausgangsverbindung ausgeht, in der $R^1$ und $R^2$ verschieden sind und $R^1$ Alkanoyl, Benzyl, Allyl oder Trityl und $R^2$ Alkanoyl, Benzyl oder Allyl bedeutet, wobei ein Rest $R^1$ oder $R^2$ eine Alkanoylgruppe ist, eine Benzyl-, Allyl- oder Tritylgruppe $R^1$ oder $R^2$ abspaltet, die freiwerdende Hydroxylgruppe acyliert und danach mit Lithiumbromid demethyliert, oder

c) zur Herstellung von Monoester-phospholipiden von einer Ausgangsverbindung ausgeht, in der $R^1$ und $R^2$ verschieden sind und $R^1$ Alkanoyl, Benzyl, Allyl oder Trityl bedeutet, $R^2$ Alkanoyl, Benzyl oder Allyl, wobei ein Rest $R^1$ oder $R^2$ eine Alkanoylgruppe ist, und mit Lithiumbromid demethyliert, oder

d) zur Herstellung von Monoäther-phospholipiden von einer Verbindung ausgeht, in der $R^1$ und $R^2$ verschieden sind und $R^1$ Alkyl, Benzyl, Allyl oder Trityl bedeutet, $R^2$ Alkyl, Benzyl oder Allyl, wobei ein Rest $R^1$ oder $R^2$ eine Alkylgruppe ist, und mit Lithiumbromid demethyliert, oder

e) zur Herstellung von Äther/Ester-phospholipiden von einer Verbindung ausgeht, in der $R^1$ und $R^2$ verschieden sind und $R^1$ Alkyl, Benzyl, Allyl oder Trityl bedeutet, $R^2$ Alkyl, Benzyl oder Allyl, wobei ein Rest $R^1$ oder $R^2$ eine Alkylgruppe ist, eine Benzyl-, Allyl- oder Tritylgruppe $R^1$ oder $R^2$ abspaltet, die entstehende Hydroxygruppe acyliert und danach mit Lithiumbromid demethyliert, oder

f) zur Herstellung von Diäther-phospholipiden von einer Verbindung ausgeht, in der $R^1$ und $R^2$ Alkyl sind, und mit Lithiumbromid demethyliert,

und wenn erforderlich oder erwünscht, in einer nach a) bis f) erhaltenen Verbindung auf an sich bekannte Weise vorhandene Schutzgruppen abspaltet und/oder den Phosphor enthaltenden Rest aminiert oder in einen anderen Phosphor enthaltenden Rest überführt, wobei die Abspaltung von Benzyl, Allyl oder Trityl auch nach der Aminierung oder Überführung in einen anderen Phosphor enthaltenden Rest erfolgen kann.

5. Arzneimittel enthaltend eine oder mehrere Verbindungen der Formel I oder II nach einem der Ansprüche 1 bis 3.

6. Arzneimittel nach Anspruch 5, dadurch gekennzeichnet, daß sie als Wirkstoff eine Mischung aus ca. 80-95 Gew.-% einer 1-Alkyl-2-acylverbindung nach Anspruch 2 und ca. 20-5 Gew.-% einer 1-Alkyl-2-OH-Verbindung nach Anspruch 3 enthalten.

**Claims**

1. Glycerol derivatives of the formulae I, II and III

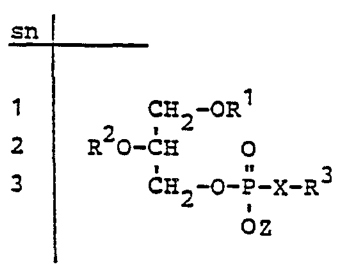

(I)

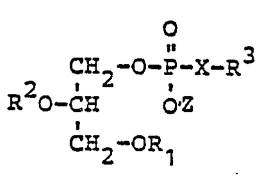

(II)

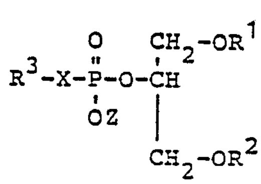

(III)

wherein Z is methyl, alkyl with a C—C multiple bond in the β-position, possibly containing a phenyl radical, benzyl or an equivalent of a physiologically compatible cation, $R^1$ and $R^2$ are the same or different and signify a hydrogen atom, the radical R or the acyl radical —COR′, in the 1- or 3-position also trityl, R signifies alkyl with 1 to 24 carbon atoms or halo-, hydroxy- or carboxyalkyl with 2 to 24 carbon atoms and R′ alkyl with 9 to 24 carbon atoms or halogen-, hydroxyl- or carboxyalkyl with 2 to 24 carbon atoms,

whereby alkyl can be straight-chained or branched and can contain double and triple bonds, and can be substituted by cycloalkyl with 3 to 6 carbon atoms or by aryl radicals, and wherein a hydroxyl and/or carboxy group can also contain a protective group, X possesses the meaning O, NH or $N(R^3)$, and $R^3$, when X = O, signifies alkyl with 1 to 20 carbon atoms or halo-, amino-, methylamino-, dimethylamino, trimethylammonio-, hydroxyl- or carboxyalkyl with 2 to 20 carbon atoms in formula I or II when $R^2$ is a hydrogen atom or the acyl radical —COR' and Z is a cation, but does not signify haloalkyl, whereby alkyl is straight-chained or branched and can contain double or triple bonds, and can be substituted by cycloalkyl with 3 to 6 carbon atoms or by aryl radicals, wherein an amino, hydroxyl and/or carboxy group can also contain a protective group, or D- or L-2-tert.-butyloxycarbonylamino-2-tert.-butyloxycarbonylethyl, 1,2- or 2,3-isopropylidenedioxypropyl, 1,2-, 1,3- or 2,3-dibenzyloxypropyl, 1,2,3,4,5-pentabenzyloxycyclohexyl or N-alkylaminoalkyl with 2 to 14 carbon atoms in the alkyl radicals, wherein the amino groups can also contain protective groups, when X = NH, alkyl with 1 to 20 carbon atoms or halo-, amino-, methylamino-, dimethylamino-, trimethylammonio-, hydroxy- or carboxyalkyl with 2 to 12 carbon atoms, whereby alkyl is straight-chained or branched and can contain double or triple bonds, and can be substituted by cycloalkyl with 3 to 6 carbon atoms or by aryl radicals, or signifies 1,2-dihydroxypropyl, wherein an amino, hydroxyl and/or carboxy group can also contain a protective group, and, when $X = N(R^3)$, is the same or different and signifies hydrogen, alkyl with 1 to 20 carbon atoms or haloalkyl with 1 to 12 carbon atoms, wherein an alkyl group is straight-chained or branched and can contain double or triple bonds and can be substituted by cycloalkyl with 3 to 6 carbon atoms or by aryl radicals, with the proviso that in formula I or II Z does not signify methyl when $R^1$ and $R^2$ signify an unsubstituted alkyl radical and excluding the sodium salt of 1-stearoyl-2-myristoyl-Sn-glycero-3-phosphocholine ($Z = Na^+$).

2. Glycerol derivatives of formula I or II according to claim 1, characterised in that $R^1$ signifies the radical $-(CH_2)_m-CH_3$, wherein m is 13-19, and $R^2$ an acyl radical COR'.

3. Glycerol derivatives of formula I or II according to claim 1, characterised in that $R^1$ signifies the radical $-(CH_2)_m-CH_3$, wherein m is 13-19, $R^2$ a hydrogen atom and, when X = O, $R^3$ signifies alkyl with 1 to 12 carbon atoms, alkyl with 2 to 11 carbon atoms ω-substituted by hydroxyl or trityloxy, alkyl with 1 to 11 carbon atoms ω-substituted by carboxy or tert.-butoxycarbonyl, 2-(amino- or tert.-butyloxycarbonylamino)-2-tert.-butyloxycarbonylethyl, 1,2-dihydroxy- or 1,2-isopropylidenedioxy-propyl, pentahydroxy- or pentabenzyloxy-cyclohexyl, or alkyl with 3-11 carbon atoms ω-substituted by amino, methylamino, dimethylamino, trimethylammonio, tert.-butyloxycarbonylamino, tert.-butyloxycarbonylmethylamino, when X = NH, $R^3$ signifies alkyl with 1 to 12 carbon atoms, alkyl with 2-11 carbon atoms ω-substituted by bromine, hydroxyl, trityloxy, amino, methylamino, dimethylamino or trimethylammonio, alkyl with 1 to 11 carbon atoms ω-substituted by carboxy or tert.-butyloxycarbonyl or 1,2-dihydroxy- or 1,2-isopropylidenedioxy-propyl and, when $X = N(R^3)$, $R^3$ is different or the same and signifies alkyl with 2 to 4 carbon atoms or ω-chloroalkyl with 1-4 carbon atoms.

4. Process for the further working up of compounds of the formulae I, II or III according to one of claims 1 to 3 for the preparation of phospholipids, characterised in that one

a) for the preparation of diester phospholipids with the same acyl radicals, starts from a compound in which $R^1$ and $R^2$ = hydrogen, acylates this and thereafter demethylates with lithium bromide, or

b) for the preparation of diester phospholipids with different acyl radicals, starts from a starting compound in which $R^1$ and $R^2$ are different and $R^1$ signifies alkanoyl, benzyl, allyl or trityl and $R^2$ alkanoyl, benzyl or allyl, whereby one radical $R^1$ or $R^2$ is an alkanoyl group, splits off a benzyl, allyl or trityl group $R^1$ or $R^2$, acylates the liberated hydroxyl group and thereafter demethylates with lithium bromide, or

c) for the preparation of monoester phospholipids, starts from a starting compound in which $R^1$ and $R^2$ are different and $R^1$ signifies alkanoyl, benzyl, allyl or trityl, $R^2$ alkanoyl, benzyl or allyl, whereby one radical $R^1$ or $R^2$ is an alkanoyl group, and demethylates with lithium bromide, or

d) for the preparation of monoether phospholipids, starts from a compound in which $R^1$ and $R^2$ are different and $R^1$ signifies alkyl, benzyl, allyl or trityl, $R^2$ alkyl, benzyl or allyl, whereby one radical $R^1$ or $R^2$ is an alkyl group, and demethylates with lithium bromide, or

e) for the preparation of ether/ester phospholipids, starts from a compound in which $R^1$ and $R^2$ are different and $R^1$ signifies alkyl, benzyl, allyl or trityl, $R^2$ alkyl, benzyl or allyl, whereby one radical $R^1$ or $R^2$ is an alkyl group, splits off a benzyl, allyl or trityl group $R^1$ or $R^2$, acylates the resulting hydroxyl group and thereafter demethylates with lithium bromide, or

f) for the preparation of diether phospholipids, starts from a compound in which $R^1$ and $R^2$ are alkyl and demethylates with lithium bromide,

and, if necessary or desired, in a compound obtained according to a) to f), splits off in per se known manner protective groups present and/or aminates the phosphorus-containing radical or converts into another phosphorus-containing radical, whereby the splitting off of benzyl, allyl or trityl can also take place after the amination or conversion into another phosphorus-containing radical.

5. Medicaments containing one or more compounds of the formula I or II according to one of claims 1 to 3.

6. Medicaments according to claim 5, characterised in that, as active material, they contain a mixture

of about 80-95 wt.% of a 1-alkyl-2-acyl compound according to claim 2 and about 20 to 5 wt.% of a 1-alkyl-2-OH compound according to claim 3.

## Revendications

1. Dérivés du glycérol de formules I, II et III

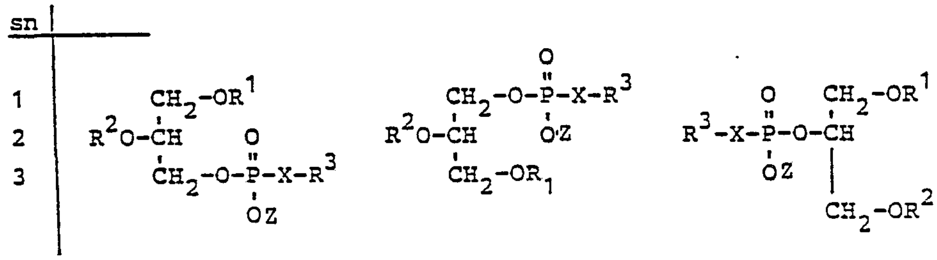

(I) (II) (III)

dans lesquelles Z représente un groupe méthyle, un groupe alkyle contenant éventuellement un groupe phényle et portant une liaison multiple C—C en position bêta, un groupe benzyle ou un équivalent d'un cation acceptable pour l'usage pharmaceutique, $R^1$ et $R^2$, ayant des significations identiques ou différentes, représentent chacun un atome d'hydrogène, le groupe R ou le radical acyle —COR', et également en position 1 ou 3 un groupe trityle, R représente un groupe alkyle en C 1-C 24 ou halogéno-, hydroxy- ou carboxy-alkyle en C 2-C 24 et R' représente un groupe alkyle en C 9-C 24 ou halogéno-, hydroxy- ou carboxy-alkyle en C 2-C 24, les groupes alkyle pouvant être à chaîne droite ou ramifiée et contenir des doubles ou triples liaisons et être substitués par des groupes cycloalkyle en C 3-C 6 ou par des groupes aryle, un groupe hydroxy et/ou carboxy pouvant également porter un groupe protecteur, X représente O, NH ou $N(R^3)$ et $R^3$, lorsque X = O, représente un groupe alkyle en C 1-C 20 ou halogéno-, amino-, méthylamino-, diméthylamino-, triméthylammonio-, hydroxy- ou carboxy-alkyle en C 2-C 20, dans la formule I ou II, lorsque $R^2$ représente un atome d'hydrogène ou le radical acyle —COR' et Z un cation, mais non un groupe halogénoalkyle, un groupe alkyle pouvant être à chaîne droite ou ramifiée et contenir des doubles ou triples liaisons et être substitué par des groupes cycloalkyle en C 3-C 6 ou par des groupes aryle, un groupe amino, hydroxy et/ou carboxy pouvant également porter un groupe protecteur, ou bien un groupe D ou L-2-tert.-butyloxycarbonylamino-2-tert.-butyloxycarbonyléthyl-, 1,2- ou 2,3-isopropylidène-dioxy-propyl-, 1,2-, 1,3- ou 2,3-dibenzyloxy-propyl-, 1,2,3,4,5-pentabenzyloxy-cyclohexyl- ou N-alkylaminoalkyle contenant 2 à 14 atomes de carbone dans les groupes alkyle, les groupes amino pouvant également porter des groupes protecteurs, lorsque X = NH, un groupe alkyle en C 1-C 20 ou halogéno-, amino-, méthylamino-, diméthylamino-, triméthylammonio-, hydroxy- ou carboxy-alkyle en C 2-C 12, les groupes alkyle pouvant être à chaine droite ou ramifiée et contenir des doubles ou triples liaisons et être substitués par un groupe cycloalkyle en C 3-C 6 ou par des groupes aryle, ou un groupe 1,2-dihydroxypropyle, un groupe amino, hydroxy et/ou carboxy pouvant également porter un groupe protecteur, et, lorsque X = $N(R^3)$, les symboles $R^3$, ayant des significations identiques ou différentes, représentent chacun l'hydrogène, un groupe alkyle en C 1-C 20 ou halogénoalkyle en C 1-C 12, un groupe alkyle étant à chaîne droite et/ou ramifiée et pouvant contenir des doubles ou triples liaisons et être substitué par un groupe cycloalkyle en C 3-C 6 ou par des groupes aryle, avec la restriction que, dans la formule I ou II, Z ne peut représenter un groupe méthyle lorsque $R^1$ et $R^2$ représentent un groupe alkyle non substitué, et à l'exception du sel de sodium de la 1-stéaroyl-2-myristoyl-sn-glycéro-3-phosphocholine ($Z = Na^+$).

2. Dérivés du glycérol de formule I ou II selon la revendication 1, caractérisés en ce que $R^1$ représente le groupe $—(CH_2)_m—CH_3$ dans lequel m a une valeur de 13 à 19 et $R^2$ représente un radical acyle COR'.

3. Dérivés du glycérol de formule I ou II selon la revendication 1, caractérisés en ce que $R^1$ représente le groupe $—(CH_2)_m—CH_3$ dans lequel m a une valeur de 13 à 19, $R^2$ représente un atome d'hydrogène et, lorsque X = O, $R^3$ représente un groupe alkyle en C 1-C 12, un groupe alkyle en C 2-C 11 substitué en oméga par un groupe hydroxy ou trityloxy, un groupe alkyle en C 1-C 11 substitué en oméga par un groupe carboxy ou tert.-butyloxycarbonyle, un groupe 2-(amino- ou tert.-butyloxycarbonylamino)-2-tert.-butyloxycarbonyl-éthyle, 1,2-dihydroxy- ou 1,2-isopropylidène-dioxy-propyle, pentahydroxy- ou pentabensyloxy-cyclohexyle, ou un groupe alkyle en C 3-C 11 substitué en oméga par un groupe amino, méthylamino, diméthylamino, triméthylammonio, tert.-butyloxycarbonylamino, tert.-butyloxycarbonylméthylamino, lorsque X = NH, $R^3$ représente un groupe alkyle en C 1-C 12, un groupe alkyle en C 2-C 11 substitué en oméga par le brome, un groupe hydroxy, trityloxy, amino, méthylamino, diméthylamino ou triméthylammonio, un groupe alkyle en C 1-C 11 substitué en oméga par un groupe carboxy ou tert.-butyloxycarbonyle ou un groupe 1,2-dihydroxy- ou 1,2-isopropylidène-dioxy-propyle et, lorsque X = $N(R^3)$, les symboles $R^3$, ayant des significations identiques ou différentes, représentent des groupes

alkyle en C 2-C 4 ou oméga-chloralkyle en C 1-C 4.

4. Procédé de préparation de phospholipides par conversion des composés de formule I, II ou III selon l'une des revendications 1 à 3, caractérisé en ce que :

a) pour préparer les diester-phospholipides à radicaux acyle identiques, on part d'un composé dans lequel $R^1$ et $R^2$ représentent l'hydrogène, on acyle ce composé puis on le déméthyle par le bromure de lithium, ou bien

b) pour préparer les diester-phospholipides à radicaux acyle différents, on part d'un composé dans lequel $R^1$ et $R^2$ sont différents et $R^1$ représente un groupe alcanoyle, benzyle, allyle ou trityle et $R^2$ représente un groupe alcanoyle, benzyle ou allyle, l'un des symboles $R^1$ et $R^2$ représentant un groupe alcanoyle, on élimine un groupe benzyle, allyle ou trityle $R^1$ ou $R^2$, on acyle le groupe hydroxy libéré puis on déméthyle par le bromure de lithium, ou bien

c) pour préparer les monoester-phospholipides, on part d'un composé dans lequel $R^1$ et $R^2$ sont différents et $R^1$ représente un groupe alcanoyle, benzyle, allyle ou trityle, $R^2$ un groupe alcanoyle, benzyle ou allyle, l'un des symboles $R^1$ et $R^2$ représentant un groupe alcanoyle, et on déméthyle par le bromure de lithium, ou bien

d) pour préparer les monoéther-phospholipides, on part d'un composé dans lequel $R^1$ et $R^2$ sont différents et $R^1$ représente un groupe alkyle, benzyle, allyle ou trityle, $R^2$ un groupe alkyle, benzyle ou allyle, l'un des symboles $R^1$ ou $R^2$ représentant un groupe alkyle, et on déméthyle par le bromure de lithium, ou bien

e) pour préparer les éther/ester-phospholipides, on part d'un composé dans lequel $R^1$ et $R^2$ sont différents et $R^1$ représente un groupe alkyle, benzyle, allyle ou trityle, $R^2$ un groupe alkyle, benzyle ou allyle, l'un des symboles $R^1$ ou $R^2$ représentant un groupe alkyle, on élimine un groupe benzyle, allyle ou trityle $R^1$ ou $R^2$, on acyle le groupe hydroxy libéré puis on déméthyle par le bromure de lithium, ou bien

f) pour préparer les diéther-phospholipides, on part d'un composé dans lequel $R^1$ et $R^2$ représentent des groupes alkyle, et on déméthyle par le bromure de lithium

et lorsque c'est nécessaire ou si on le désire, on élimine de manière connue en soi les groupes protecteurs présents dans un composé obtenu selon a) à f) et/ou on amine le radical contenant du phosphore ou bien on le convertit en un autre radical contenant du phosphore, l'élimination du groupe benzyle, allyle ou trityle pouvant également être effectuée après l'amination ou la conversion du radical contenant le phosphore en un autre radical contenant du phosphore.

5. Médicaments contenant un ou plusieurs composés de formule I ou II selon l'une des revendications 1 à 3.

6. Médicaments selon la revendication 5, caractérisés en ce qu'ils contiennent en tant que substance active un mélange d'environ 80 à 95 % en poids d'un composé 1-alkyl-2-acylé selon la revendication 2 et environ 20 à 5 % en poids d'un composé en 1-alkyl-2-OH selon la revendication 3.